# EUROPEAN PATENT APPLICATION

(11) **EP 4 421 096 A1**
(43) Date of publication of application: **28.08.2024**
(21) Application number: 22883427.1
(22) Date of filing: 12.10.2022
(51) Int. Cl.: C08B 37/00, A61K 8/31, A61K 8/73, A61K 8/89, A61Q 19/00

(54) **SILICONE-MODIFIED PULLULAN, COMPOSITION INCLUDING SAID MODIFIED PULLULAN, AND COSMETIC**

(30) Priority: 22.10.2021 JP 2021173111
(71) Applicant: SHIN-ETSU CHEMICAL CO., LTD., Tokyo 1000005 (JP)
(72) Inventor: MORIYA, Hiroyuki, Annaka-shi, Gunma 379-0224 (JP)
(74) Representative: V.O.
(86) International application number: PCT/JP2022/037985
(87) International publication number: WO 2023/068123

(57) **Abstract**

The present invention provides a silicone-modified pullulan, the silicone-modified pullulan being an addition reaction product of the following components (a) and (b): (a) pullulan having a water content of 1.0 mass% or less and a calcium content of less than 100 ppm; and (b) isocyanate group-containing silicone represented by formula (1), wherein the silicone-modified pullulan has a calcium content of less than 100 ppm and a content of the urea group-containing silicone represented by formula (2) of less than 2,000 ppm.

## Description

### TECHNICAL FIELD

The present invention relates to a silicone-modified pullulan, a composition comprising the modified pullulan, and a cosmetic. More specifically, the present invention relates to a silicone-modified pullulan with a reduced amount of impurities, and a method for preparing the silicone-modified pullulan.

### BACKGROUND OF THE INVENTION

Silicone-modified polysaccharides, which are polysaccharides modified with silicone, have conventionally been examined (Patent Literature 1 and 2). For example, Patent Literature 1 discloses a siloxane-containing cellulose derivative having the properties of both cellulose and silicone. Patent Literature 2 discloses siloxane-containing pullulan having the properties of both pullulan and a silicone compound. Further, adding silicone-modified polysaccharides to cosmetics is also being considered (Patent Literature 3 to 6). Patent Literature 3 discloses a sunscreen cosmetic characterized by using a specific siliconized polysaccharide compound in combination with an organic ultraviolet light absorber. Patent Literature 4 discloses a water-in-oil emulsified cosmetic containing a siliconized polysaccharide compound. Patent Literature 6 discloses an eye makeup composition containing siliconized pullulan. As described in Patent Literature 3 to 6, cosmetics containing silicone-modified polysaccharides have high water- and oil resistance and excellent usability.

### PRIOR LITERATURE

### PATENT LITERATURE

Patent Literature 1: Japanese Patent Application Laid-Open No. Hei 6-145201/1994
Patent Literature 2: Japanese Patent Application Laid-Open No. Hei 8-134103/1996
Patent Literature 3: Japanese Patent Application Laid-Open No. Hei 10-29921/1998
Patent Literature 4: Japanese Patent Application Laid-Open No. 2001-278729
Patent Literature 5: WO 2012/133293
Patent Literature 6: Japanese Patent Application Laid-Open No. 2017-218413

### DISCLOSURE OF THE INVENTION

### PROBLEMS TO BE SOLVED BY THE INVENTION

However, conventional silicone-modified polysaccharides have had problems with storage stability when dissolved in organic oil. More specifically, their appearance would be cloudy, and needle-like crystals would sometimes be deposited. These are considered to be due to residual ionic metal salts and the precipitation of unreacted dimerized monomers. Accordingly, there has been a demand for silicone-modified pullulan that is stable when formed into compositions. In addition, unreacted monomers have isocyanate groups, so that there is a concern that irritation to the skin may occur if a large amount of the unreacted monomers are remaining.

### SOLUTIONS TO THE PROBLEMS

The present invention was made in view of the aforesaid problems, and one of the objects of the present invention is to provide a silicone-modified pullulan-containing composition with improved stability comprising silicone-modified pullulan and an oil agent, and a method for preparing the same. In particular, an object of the present invention is to provide a composition comprising silicone-modified pullulan with a small amount of impurities, having high storage stability, and not causing deterioration of usability when mixed into cosmetics. Such a silicone-modified pullulan-containing composition provides a composition with high safety and high transparency.

Pullulan is generally produced by culturing pullulan-producing bacteria, and ionic inorganic salts are used in the culture medium. As a result of extensive research on the conventional problems, the present inventor has found that when pullulan has an inorganic salt of calcium or the like, a composition containing silicone-modified pullulan is cloudy, and that side reactions preferentially occur when pullulan is modified with silicone. In addition, pullulan has hydroxyl groups and thus absorbs moisture over time. The side reactions by the moisture cause generation of urea group-containing silicone during the production of silicone-modified pullulan, and the removal of the urea group-containing silicone is difficult, even through a washing step. Accordingly, the present inventor has found that pullulan subjected to dehydration in advance makes it possible to suppress the generation of urea group-containing silicone during the production of silicone-modified pullulan.

Further, organic tin and amines have been conventionally used as reaction catalysts for pullulan and silicone-modified isocyanates. However, organic tin had problems for safety, and amine catalysts had problems with low catalyst activity. Therefore, the specific catalyst is used in the preparation method of the present invention and, thereby, the ratio of adding the silicone into pullulan is improved and a silicone-modified pullulan having a glass transition point of 100 to 150°C is obtained.

That is, the present invention provides a silicone-modified pullulan, the silicone-modified pullulan being an addition reaction product of the following components (a) and (b):
(a) pullulan having a water content of 1.0 mass% or less and a calcium content of less than 100 ppm; and
(b) isocyanate group-containing silicone represented by the following formula (1): 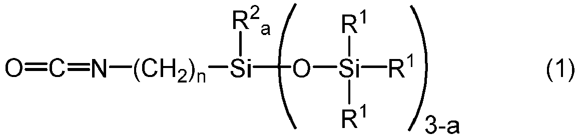 wherein R¹ is, independently of each other, a group selected from an alkyl group having 1 to 8 carbon atoms, a cycloalkyl group having 3 to 8 carbon atoms, a fluorine-substituted alkyl group having 1 to 8 carbon atoms, an aryl group having 6 to 12 carbon atoms, and an aralkyl group having 7 to 12 carbon atoms; R² is an alkyl group having 1 to 4 carbon atoms or a phenyl group; n is an integer of 1 to 10; and a is an integer of 0 to 3,

wherein the silicone-modified pullulan has a calcium content of less than 100 ppm and a content of the urea group-containing silicone represented by the following formula (2) of less than 2,000 ppm, 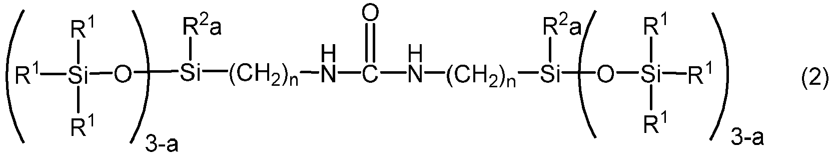
wherein R¹, R², *n* and a are as described above.

Further, the present invention provides a composition comprising the silicone-modified pullulan, a cosmetic comprising the composition, and a method for preparing the aforesaid silicone-modified pullulan.

### EFFECTS OF THE INVENTION

The silicone-modified pullulan of the present invention has a small amount of impurities, such as by-products. The silicone-modified pullulan-containing composition of the present invention has a small amount of impurities, so that the storage stability of the composition is improved and the deterioration of usability does not occur with mixing it into cosmetics. Further, the composition has high safety and transparency, and is suitably added to coating agents and paints.

### DETAILED DESCRIPTION OF THE INVENTION

The following describes the present invention in more detail.

The silicone-modified pullulan of the present invention is characterized by being an addition reaction product of the following components (a) and (b):
(a) pullulan having a water content of 1.0 mass% or less and a calcium content of less than 100 ppm; and
(b) isocyanate group-containing silicone represented by the following formula (1): 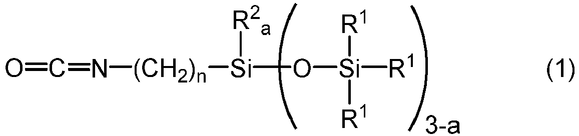 wherein R¹ is, independently of each other, a group selected from an alkyl group having 1 to 8 carbon atoms, a cycloalkyl group having 3 to 8 carbon atoms, a fluorine-substituted alkyl group having 1 to 8 carbon atoms, an aryl group having 6 to 12 carbon atoms, and an aralkyl group having 7 to 12 carbon atoms; R² is an alkyl group having 1 to 4 carbon atoms or a phenyl group; n is an integer of 1 to 10; and a is an integer of 0 to 3.

The silicone-modified pullulan of the present invention has a calcium content of less than 100 ppm and a content of the urea group-containing silicone represented by the following formula (2) of less than 2,000 ppm, 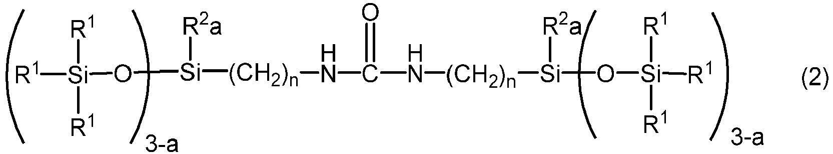 wherein R¹, R², n, and a are as described above.
More preferably, the amount of the remaining isocyanate group-containing silicone represented by the aforesaid formula (1) is less than 2,000 ppm.

In the present invention, the pullulan (a) is characterized by having a water content of 1.0 mass% or less, more preferably 0.1 mass% or less, or even more preferably 0.05 mass% or less, based on the entire amount of pullulan. The lower limit of the water content is not particularly limited, and the lower the better. The lower limit is preferably 0.001 mass% or more, or more preferably 0.005 mass% or more. If the water content exceeds the aforesaid upper limit, a large amount of the urea group-containing silicone represented by formula (2) may be produced as a by-product during the reaction with the isocyanate group-containing silicone (b), which is not preferable. The water content of the pullulan may be determined by the Karl Fischer method.

The method for adjusting the water content of the pullulan to 1.0 mass% or less is not particularly limited, but examples include a method of drying the pullulan under reduced pressure at 50 to 120°C, and a method of removing water by azeotropic dehydration with hexane, toluene, or the like.

The content of calcium contained in the pullulan of the present invention is characterized by being less than 100 ppm, preferably 50 ppm or less, more preferably 30 ppm or less, based on the entire amount of pullulan. The lower limit of the calcium content is not particularly limited, and the lower the better. The lower limit is preferably 0.01 ppm or more, or more preferably 0.1 ppm or more. If the calcium content is equal to or more than the aforesaid upper limit, a large amount of the urea group-containing silicone represented by formula (2) may be produced as a by-product during the reaction. Further, the resulting composition may have a cloudy appearance, which is not preferable. In the present invention, the calcium content refers to a calcium element equivalent value determined by Inductively coupled plasma (ICP) optical emission spectrometry.

The pullulan preferably has a weight average molecular weight of 1,000 to 5,000,000, or more preferably 30,000 to 400,000. The weight average molecular weight is determined by gel permeation chromatography (GPC) analysis, wherein the solvent is DMF; pullulan equivalent value; the measurement temperature is 25°C.

Any pullulan that satisfies the aforesaid requirements may be used in the present invention, and commercially available purified pullulan may be used. Further, commercially available pullulan that has been previously desalted and purified may be used after being dehydrated. For example, it is preferable to use pullulan (desalted and purified product) manufactured by Hayashibara Co., Ltd., which is sold for cosmetic use, after being dehydrated.

The component (b) is isocyanate group-containing silicone and is represented by the following formula (1): 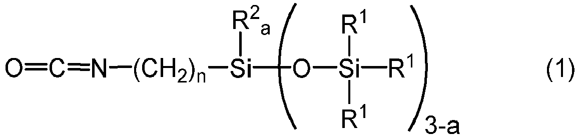 In formula (1), R¹ is, independently of each other, a group selected from an alkyl group having 1 to 8 carbon atoms, a cycloalkyl group having 3 to 8 carbon atoms, a fluorine-substituted alkyl group having 1 to 8 carbon atoms, an aryl group having 6 to 12 carbon atoms, and an aralkyl group having 7 to 12 carbon atoms. Examples of alkyl groups include methyl, ethyl, propyl, butyl, pentyl, hexyl, heptyl, and octyl groups. Examples of cycloalkyl groups include cyclopentyl and cyclohexyl groups. Examples of fluorine-substituted alkyl groups include trifluoropropyl, nonafluorohexyl, and heptadecafluorodecyl groups. Examples of aryl groups include phenyl and tolyl groups. Examples of aralkyl groups include benzyl and phenethyl groups. Preferred is an alkyl group having 1 to 8 carbon atoms or a phenyl group, or more preferred is a methyl group.

R² is an alkyl group having 1 to 4 carbon atoms or a phenyl group. Among these, preferred is a methyl group, an ethyl group, or a phenyl group. *n* is an integer of 1 to 10, preferably an integer of 2 to 8, more preferably an integer of 3 to 6, or most preferably 3. a is an integer of 0 to 3, preferably 0, 1 or 2, or most preferably 0.

Examples of the isocyanate group-containing silicone (b) include compounds represented by the following formulas:

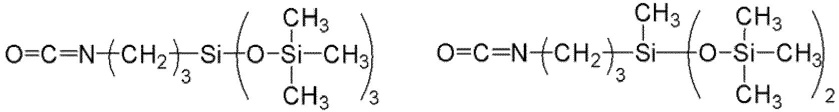

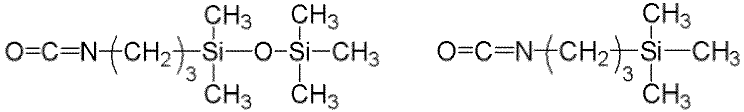

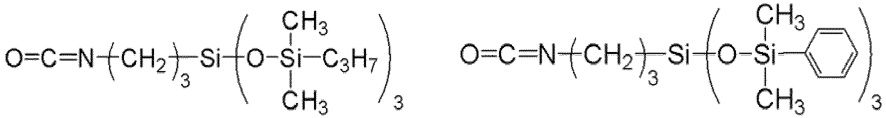

The silicone-modified pullulan of the present invention is an addition reaction product of the pullulan (a) and the isocyanate group-containing silicone (b). The mixing ratio of the pullulan (a) and the isocyanate group-containing silicone (b) in the addition reaction is such that the amount of the isocyanate group-containing silicone is preferably 500 to 800 parts by mass, or more preferably 600 to 700 parts by mass, based on 100 parts by mass of the pullulan.

The addition reaction is preferably conducted in an organic solvent (c). Examples of the organic solvent include esters, such as butyl acetate; ketones, such as methyl ethyl ketone, methyl isobutyl ketone, and cyclohexanone; aromatic hydrocarbons, such as toluene and xylene; ethers, such as dibutyl ether, tetrahydrofuran, and dioxane; and amides, such as N,N-dimethylformamide and N-methylpyrrolidone. These may be used singly or in combination of two or more. The amount of the organic solvent (c) may be adjusted appropriately, but is preferably 1,000 to 10,000 parts by mass, relative to 100 parts by mass of the pullulan. The organic solvent (c) used in the present invention preferably has a low water content. The water content of the organic solvent is preferably 5,000 ppm or less, or more preferably 1 to 1,000 ppm. When water content is within this range, the production of by-products is suppressed, which is preferable.

Although the aforesaid addition reaction generally proceeds without a catalyst, it is preferable to use a catalyst in the preparation method of the present invention. The catalyst is preferably an organometallic compound containing a metallic element, such as iron, bismuth, zirconium, titanium, aluminum, or copper, in terms of catalyst activity and safety. Organometallic compound catalysts have higher catalyst activity than amine catalysts and improves the silicone addition ratio and, thereby, silicone-modified pullulan with a reduced amount of impurities having a glass transition point of 100 to 150°C is provided. Examples of organometallic compounds include metal complexes of acetylacetone, such as aluminum acetylacetonate, iron acetylacetonate, copper acetylacetonate, titanium acetylacetonate, and zirconium acetylacetonate; or bismuth carboxylate. Particularly preferred are metal complexes of acetylacetone, and among these preferred is an iron complex of acetylacetone. The amount of the organometallic compound is 0.0001 to 0.1 parts by mass, or preferably 0.001 to 0.5 parts by mass, relative to 100 parts by mass of the pullulan. In the preparation method of the present invention, such an organometallic compound is used as a catalyst and, thereby, the addition reaction ratio is increased and the amount of impurities is reduced.

The addition reaction temperature is preferably 50 to 150°C. The reaction time may be appropriately selected. After the completion of the reaction, washing and drying are conducted, whereby silicone-modified pullulan is obtained. The silicone-modified pullulan obtained by the preparation method of the present invention is characterized by having a low amount of by-products. That is, the silicone-modified pullulan is characterized by having a calcium content of less than 100 ppm and a content of the urea group-containing silicone represented by the aforesaid formula (2) of less than 2,000 ppm. Preferably, the amount of the remaining isocyanate group-containing silicone represented by the aforesaid formula (1) used as a raw material is less than 2,000 ppm.

In the silicone-modified pullulan, the calcium content is less than 100 ppm, preferably 50 ppm or less, more preferably 10 ppm or less, or even more preferably 5 ppm or less. The lower limit is not particularly limited, and the lower the better. For example, the lower limit is 0.01 ppm or more. If the calcium content is equal to or more than the aforesaid upper limit, the resulting composition has a milky appearance, and precipitates may be formed in the composition, which impairs the aesthetics. The calcium content may be determined by the method for measuring the calcium content of pullulan described above.

In the silicone-modified pullulan, the content of the urea group-containing silicone represented by the aforesaid formula (2) is less than 2,000 ppm, preferably 1,800 ppm or less, more preferably 1,500 ppm or less, or even more preferably 1,000 ppm or less. The lower limit is not particularly limited, and the lower the better. For example, the lower limit is 0.1 ppm or more, or preferably 1 ppm or more. If the content is equal to or more than the aforesaid upper limit, the appearance becomes milky, and precipitates may be deposited over time. The removal of the urea group-containing silicone by washing is difficult. The content of the urea group-containing silicone may be determined by gas chromatography.

Further, in the silicone-modified pullulan, the amount of the remaining isocyanate group-containing silicone represented by the aforesaid formula (1) used as a raw material is preferably less than 2,000 ppm, more preferably 1,800 ppm or less, even more preferably 1,500 ppm or less, or still even more preferably 1,000 ppm or less. The lower limit is not particularly limited, and the lower the better. For example, the lower limit is 0.1 ppm or more, or preferably 1 ppm or more. If the remaining amount is equal to or more than the aforesaid upper limit, precipitates may be deposited in the composition over time. In addition, mixing into cosmetics in that case is not preferable for safety reasons. The amount of the remaining isocyanate group-containing silicone may be determined by headspace gas chromatography.

The silicone-modified pullulan of the present invention preferably has a glass transition point of 100 to 150°C, or more preferably 110 to 140°C. When the glass transition point is within this range, a tough and flexible film is formed. Accordingly, when the silicone-modified pullulan is used in cosmetics, the cosmetic does not irritate the skin and is long-lasting, which is preferable. The glass transition point in the present invention is the value determined by a dynamic viscoelasticity measurement.

The present invention further provides a composition comprising the silicone-modified pullulan and various oil agents. Preferably the present invention provides a silicone-modified pullulan composition comprising:
(A) 20 to 50 mass% of the silicone-modified pullulan, and
(B) 50 to 80 mass% of one or more oil agents selected from a silicone oil, a hydrocarbon oil, and an ester oil.

Examples of the silicone oil in the component (B) include low-viscosity to high-viscosity linear or branched organopolysiloxanes, such as dimethylpolysiloxane, tristrimethylsiloxymethylsilane, caprylylmethicone, phenyltrimethicone, tetrakistrimethylsiloxysilane, methylphenylpolysiloxane, methylhexylpolysiloxane, methylhydrogenpolysiloxane, and dimethylsiloxane-methylphenylsiloxane copolymer. Other examples include cyclic organopolysiloxanes, such as octamethylcyclotetrasiloxane, decamethylcyclopentasiloxane, dodecamethylcyclohexasiloxane, tetramethyltetrahydrogencyclotetrasiloxane, and tetramethyltetraphenylcyclotetrasiloxane; and amino-modified organopolysiloxane, pyrrolidone-modified organopolysiloxane, pyrrolidone carboxylic acid-modified organopolysiloxane; other silicone rubbers, such as gum-like dimethylpolysiloxane with a high degree of polymerization, gum-like amino-modified organopolysiloxane, and gum-like dimethylsiloxane-methylphenylsiloxane copolymer; and cyclic organopolysiloxane solutions of silicone gum or rubber.

Examples of hydrocarbon oils include linear, branched, and volatile hydrocarbon oils and the like. Specific examples include ozokerite, α-olefin oligomer, light isoparaffin, isododecane, isohexadecane, light liquid isoparaffin, squalane, synthetic squalane, vegetable squalane, squalene, ceresin, paraffin, paraffin wax, polyethylene wax, polyethylene-polypropylene wax, (ethylene/propylene/styrene) copolymer, (butylene/propylene/styrene) copolymer, liquid paraffin, liquid isoparaffin, pristane, polyisobutylene, hydrogenated polyisobutene, microcrystalline wax, petrolatum, and the like. Examples of higher fatty acids include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, 12-hydroxystearic acid, and the like.

Examples of ester oils include diisobutyl adipate, 2-hexyldecyl adipate, di-2-heptylundecyl adipate, N-alkyl glycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dioctanoate, neopentyl glycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isononyl isononanoate, isotridecyl isononanoate, isopropyl palmitate, 2-ethylhexyl palmitate, 2-hexyldecyl palmitate, 2-heptylundecyl palmitate, cholesteryl 12-hydroxystearate, dipentaerythritol fatty acid ester, isopropyl myristate, octyldodecyl myristate, 2-hexyldecyl myristate, myristyl myristate, hexyldecyl dimethyloctanoate, ethyl laurate, hexyl laurate, N-lauroyl-L-glutamic acid-2-octyldodecyl ester, lauroyl sarcosine isopropyl ester, diisostearyl malate, and the like. Examples of glyceride oils include acetoglyceryl, glyceryl triisooctanoate, glyceryl triisostearate, glyceryl triisopalmitate, glyceryl tribehenate, glyceryl monostearate, glyceryl di-2-heptylundecanoate, glyceryl trimyristate, diglyceryl myristate isostearate, and the like.

The silicone-modified pullulan composition of the present invention preferably has a transparent appearance and a viscosity at 25°C of 50 to 200,000 mPa·s as determined by the rotational viscosity measurement method described in the Japanese Standards of Quasi-drug Ingredients 2006.

It is preferable for the silicone-modified pullulan composition that white precipitates are generated when 1 part by mass of the composition consisting of 30 mass% of the silicone-modified pullulan and 70 mass% of hydrocarbon oil, such as isododecane, is mixed with 1.5 parts by mass of acetone. If the composition is dissolved without generating white precipitates in the mixing test with acetone, the stability of the composition over time and the stability when mixed into cosmetics or coating agents may deteriorate. The silicone-modified pullulan composition of the present invention is modified with silicone so as to have a glass transition point of 100 to 150°C, thereby generating white precipitates when mixed with acetone.

The silicone-modified pullulan composition of the present invention may be added to cosmetics. The amount of the silicone-modified pullulan composition is not particularly limited, but is suitably added within the range of 0.05 to 20 mass%, or preferably 0.1 to 10 mass%, of the entire cosmetic.

Cosmetics optionally contain cosmetically acceptable ingredients. Their forms include powders, oils, water-in-oil emulsions, oil-in-water emulsions, non-aqueous emulsions, and multi-emulsions such as W/O/W and O/W/O. Examples of cosmetics include skin care cosmetics, such as toners, lotions, creams, makeup removers, facial masks, liquid oils, cosmetics for massages, beauty serums, beauty oils, cleansers, deodorants, hand creams, lip balms, and wrinkle concealers; makeup cosmetics, such as makeup bases, concealers, face powders, powder foundations, liquid foundations, cream foundations, oil foundations, blushes, eye shadows, mascara, eyeliners, eyebrow makeup, and lipsticks; hair cosmetics, such as shampoos, conditioners, hair treatments, and styling products; antiperspirants; UV protection cosmetic compositions, such as sunscreen oils, sunscreen lotions, and sunscreen creams; and the like. Cosmetics may have various forms, such as liquid, emulsion, cream, solid, paste, gel, powder, pressed, multilayered, mousse, spray, stick, and pencil forms.

### EXAMPLES

The present invention will be explained below in further detail with reference to a series of Examples and Comparative Examples, though the present invention is in no way limited by these Examples.

In the following, the pullulan used in Examples 1 and 2 is a cosmetic pullulan manufactured by Hayashibara Co., Ltd., which is a desalted and purified product with a weight average molecular weight of 200,000. The pullulan used in Comparative Examples 1 and 2 is Pullulan PF-20 manufactured by Hayashibara Co., Ltd., which is a non-desalted and purified product with a weight average molecular weight of 200,000.

### [Example 1]

In a 7,000-ml reactor, were placed 100 g of pullulan and 1,300 g of hexane, and the mixture was azeotropically dehydrated under reduced pressure while being stirred at 80°C for 2 hours. The pullulan had a water content of 200 ppm and a calcium content of 10 ppm. 3,000 g of N-methylpyrrolidone and 0.03 g of an iron complex of acetylacetone were added thereto and dissolved by heating at 120°C. 560 grams of the isocyanate group-containing organopolysiloxane represented by formula (3) were added dropwise thereto. After stirring and reacting at 120°C for 5 hours, 2,600 g of N-methylpyrrolidone separated from the reaction liquid was extracted, and 2,300 g of hexane was added to the remaining resin, followed by stirring and dissolution. 1,200 grams of methanol were added thereto, followed by stirring, and the methanol layer was discarded. After repeating this washing process twice, drying under reduced pressure was conducted at 80°C for 8 hours, and 525 g of silicone-modified pullulan was taken out. The silicone-modified pullulan had a glass transition point of 125°C.

In the obtained silicone-modified pullulan, the calcium content was less than 1 ppm, the content of the isocyanate group-containing organopolysiloxane represented by the following formula (3) was 200 ppm, and the content of the urea group-containing organopolysiloxane represented by the following formula (4) was 50 ppm.

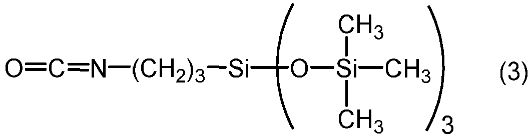

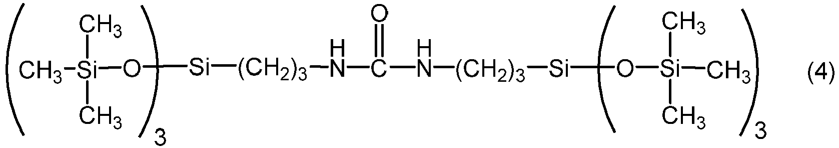

30 grams of the obtained silicone-modified pullulan and 70 g of isododecane were mixed and dissolved to obtain a pale yellow, transparent composition with a viscosity of 450 mPa·s. When 1 g of the composition and 1.5 g of acetone were mixed, white precipitates were deposited. The appearance of the composition did not change, even after storing it for one month at room temperature.

### [Example 2]

In a 5,000-ml reactor, were placed 100 g of pullulan and 400 g of hexane, and the mixture was azeotropically dehydrated under reduced pressure while being stirred at 80°C for 2 hours. The pullulan had a water content of 400 ppm and a calcium content of 10 ppm. 1,500 g of N-methylpyrrolidone and 0.05 g of bismuth carboxylate (product name: K-KAT XK-640; manufactured by King Industry) were added thereto and dissolved by heating at 120°C. 600 grams of the isocyanate group-containing organopolysiloxane represented by formula (3) were added dropwise thereto. After stirring and reacting at 120°C for 5 hours, 1,000 g of methyl ethyl ketone was added, followed by stirring and dissolution. This solution was added to 3,000 g of methanol, followed by stirring to precipitate resin. After washing three times with 300 g of methanol and drying under reduced pressure at 80°C for 8 hours, 600 g of resin was taken out. The obtained silicone-modified pullulan had a glass transition point of 118°C.

In the obtained silicone-modified pullulan, the calcium content was less than 1 ppm, the content of the isocyanate group-containing organopolysiloxane represented by formula (3) was 1,800 ppm, and the content of the urea group-containing organopolysiloxane represented by formula (4) was 1,500 ppm.

30 grams of the obtained silicone-modified pullulan and 70 g of isododecane were mixed and dissolved to obtain a pale yellow, transparent composition with a viscosity of 750 mPa·s. When 1 g of the composition and 1.5 g of acetone were mixed, white precipitates were deposited. The appearance of the composition did not change, even after storing for one month at room temperature.

### [Comparative Example 1]

In a 7,000-ml reactor, was placed 100 g of pullulan having a water content of 3.5% and a calcium content of 1,200 ppm. 3,000 g of N-methylpyrrolidone and 10 g of triethylamine were added thereto and dissolved by heating at 120°C. 560 grams of the isocyanate group-containing organopolysiloxane represented by formula (3) were added dropwise thereto. After stirring and reacting at 120°C for 5 hours, 1,800 g of N-methylpyrrolidone separated from the reaction liquid was extracted, and 2,300 g of hexane was added to the remaining resin, followed by stirring and dissolution. 1,200 grams of methanol were added thereto, followed by stirring, and the methanol layer was discarded. After repeating this washing process twice, drying under reduced pressure was conducted at 80°C for 8 hours, and 470 g of resin was taken out. The obtained silicone-modified pullulan had a glass transition point of 155°C.

In the obtained silicone-modified pullulan, the calcium content was 700 ppm, the content of the isocyanate group-containing organopolysiloxane represented by formula (3) was 7,000 ppm, and the content of the urea group-containing organopolysiloxane represented by formula (4) was 8,000 ppm.

30 grams of the obtained silicone-modified pullulan and 70 g of isododecane were mixed and dissolved to obtain a pale yellow, milky composition with a viscosity of 1,400 mPa·s. When 1 g of the composition and 1.5 g of acetone were mixed, white precipitates were not generated, and the mixture was dissolved to produce a transparent solution. White precipitates were generated in the composition after storing it for one month at room temperature, and the stability over time was inferior.

### [Comparative Example 2]

In a 7,000-ml reactor, was placed 100 g of pullulan which had previously been dried at 110°C for 2 hours. The pullulan had a water content of 1.2% and a calcium content of 1,200 ppm. 3,000 g of N-methylpyrrolidone and 10 g of triethylamine were added thereto and dissolved by heating at 120°C. 560 grams of the isocyanate group-containing organopolysiloxane represented by formula (3) were added dropwise thereto. After stirring and reacting at 120°C for 5 hours, 1,800 g of N-methylpyrrolidone separated from the reaction liquid was extracted, and 2,300 g of hexane was added to the remaining resin, followed by stirring and dissolution. 1,200 grams of methanol were added thereto, followed by stirring, and the methanol layer was discarded. After repeating this washing process twice, drying under reduced pressure was conducted at 80°C for 8 hours, and 480 g of resin was taken out. The obtained silicone-modified pullulan had a glass transition point of 155°C.

In the obtained silicone-modified pullulan, the calcium content was 700 ppm, the content of the isocyanate group-containing organopolysiloxane represented by formula (3) was 6,000 ppm, and the content of the urea group-containing organopolysiloxane represented by formula (4) was 7,000 ppm.

30 grams of the obtained silicone-modified pullulan and 70 g of isododecane were mixed and dissolved to obtain a pale yellow, milky composition with a viscosity of 1,200 mPa·s. When 1 g of the composition and 1.5 g of acetone were mixed, white precipitates were not generated, and the mixture was dissolved to produce a transparent solution. White precipitates were generated in the composition after storing it for one month at room temperature, and the stability over time was inferior.

The preparation method of the present invention provides silicone-modified pullulan with a small amount of impurities, such as by-products. The composition comprising the silicone-modified pullulan of the present invention has improved storage stability and does not cause deterioration of usability when mixed into cosmetics. Further, the present composition has high safety and transparency, and is suitably added to coating agents and paints.

## Claims

1. A silicone-modified pullulan, the silicone-modified pullulan being an addition reaction product of the following components (a) and (b):
(a) pullulan having a water content of 1.0 mass% or less and a calcium content of less than 100 ppm; and
(b) an isocyanate group-containing silicone represented by the following formula (1): 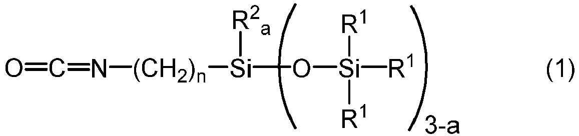
wherein R¹ is, independently of each other, a group selected from an alkyl group having 1 to 8 carbon atoms, a cycloalkyl group having 3 to 8 carbon atoms, a fluorine-substituted alkyl group having 1 to 8 carbon atoms, an aryl group having 6 to 12 carbon atoms, and an aralkyl group having 7 to 12 carbon atoms; R² is an alkyl group having 1 to 4 carbon atoms or a phenyl group; n is an integer of 1 to 10; and a is an integer of 0 to 3,
wherein the silicone-modified pullulan has a calcium content of less than 100 ppm and a content of the urea group-containing silicone represented by the following formula (2) of less than 2,000 ppm, 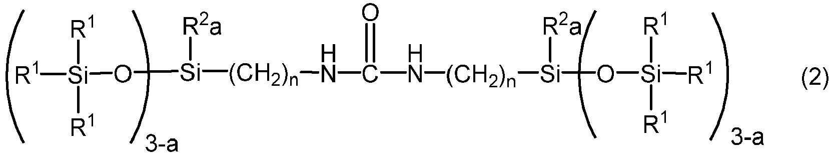
wherein R¹, R², *n* and *a* are as described above.

2. The silicone-modified pullulan according to claim 1, wherein the silicone-modified pullulan has a glass transition point of 100 to 150°C as determined by a dynamic viscoelasticity measurement.

3. The silicone-modified pullulan according to claim 1 or claim 2, wherein the amount of the remaining isocyanate group-containing silicone represented by the aforesaid formula (1) contained in the silicone-modified pullulan is less than 2,000 ppm.

4. A composition comprising
(A) 20 to 50 mass% of the silicone-modified pullulan according to any one of claims 1 to 3, and
(B) 50 to 80 mass% of one or more oil agents selected from a silicone oil, a hydrocarbon oil, and an ester oil.

5. The composition according to claim 4, wherein the composition has a viscosity at 25°C of 50 to 200,000 mPa·s as determined according to the Japanese Industrial Standards K7117-1:1999.

6. A cosmetic comprising the composition according to claim 4 or claim 5.

7. A method for preparing a silicone-modified pullulan, wherein the method comprises steps of subjecting
(a) pullulan having a water content of 1.0 mass% or less and a calcium content of less than 100 ppm; and
(b) an isocyanate group-containing silicone represented by the following formula (1): 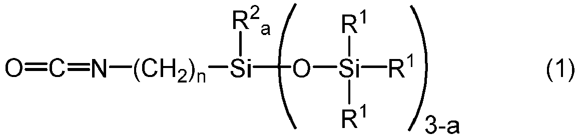
wherein R¹ is, independently of each other, a group selected from an alkyl group having 1 to 8 carbon atoms, a cycloalkyl group having 3 to 8 carbon atoms, a fluorine-substituted alkyl group having 1 to 8 carbon atoms, an aryl group having 6 to 12 carbon atoms, and an aralkyl group having 7 to 12 carbon atoms; R² is an alkyl group having 1 to 4 carbon atoms or a phenyl group; n is an integer of 1 to 10; and a is an integer of 0 to 3,
to an addition reaction to thereby obtain the silicone-modified pullulan,
wherein the silicone-modified pullulan has a calcium content of less than 100 ppm and a content of the urea group-containing silicone represented by the following formula (2) of less than 2,000 ppm, 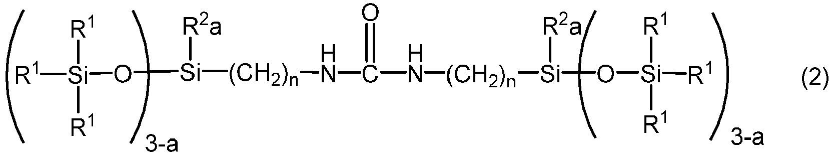
wherein R¹, R², n and a are as described above.

8. The method according to claim 7, wherein the addition reaction is conducted in the presence of an organometallic compound.

9. The method according to claim 8, wherein the organometallic compound is selected from metal complexes of acetylacetone and a bismuth carboxylate.

10. The method according to any one of claims 7 to 9, wherein the amount of component (b) is 500 to 700 parts by mass, relative to 100 parts by mass of component (a).

11. The method according to any one of claims 7 to 10, wherein the addition reaction is conducted in an organic solvent and the amount of the organic solvent is 1,000 to 10,000 parts by mass, relative to 100 parts by mass of component (a).

12. The method according to any one of claims 7 to 11, wherein an amount of the remaining isocyanate group-containing silicone represented by the aforesaid formula (1) contained in the silicone-modified pullulan is less than 2,000 ppm.

13. The method according to any one of claims 7 to 12, wherein the silicone-modified pullulan has a glass transition point of 100 to 150°C as determined by a dynamic viscoelasticity measurement.
